# EUROPEAN PATENT APPLICATION

(11) **EP 3 851 532 A1**
(43) Date of publication of application: **21.07.2021**
(21) Application number: 20382027.9
(22) Date of filing: 20.01.2020
(51) Int. Cl.: C12N 15/113, C12N 15/63

(54) **GENE EDITING FOR THE TREATMENT OF EPIDERMOLYSIS BULLOSA**

(71) Applicant: Universidad Carlos III de Madrid, 28919 Leganés - Madrid (ES); Centro de Investigaciones Energéticas, Medio Ambientales y Technológicas, O.A., M.P., 28040 Madrid (ES); Consorcio Centro de Investigación Biomédica en Red M.P., 28029 Madrid (ES); Fundación Instituto Investigación Sanitaria Jiménez Díaz, 28040 Madrid (ES)
(72) Inventor: Bonafont Aragó, José, 28919 Leganés (Madrid) (ES); Larcher Laguzzi, Fernando, 28040 Madrid (ES); Murillas Angoiti, Rodolfo, 28040 Madrid (ES); del Río Nechaevsky, Marcela, 28919 Leganés (Madrid) (ES); Mencía Rodriguez, Ángeles, 28029 Madrid (ES); García Díez, Marta, 28919 Leganés (Madrid) (ES); Escámez Toledano, María José, 28029 Madrid (ES)
(74) Representative: Hoffmann Eitle

(57) **Abstract**

The present invention relates to the treatment of Epidermolysis Bullosa, particularly the recessive dystrophic subtype (RDEB), using the Clustered- Regularly Interspaced Short Palindromic Repeats (CRISPR) system. This technology offers the possibility to design a single guide RNA (sgRNA) which is incorporated into a CRISPR- associated protein (Cas9) to recognize and induce DNA double-strand breaks at a specific target location. DNA double-strand breaks will be repaired by homologous recombination (HR) in the presence of a donor sequence for Epidermolysis Bullosa gene repair. In the context of Epidermolysis Bullosa, this allows to repair the mutation/s causing the disease.

## Description

### Technical field of the invention

The present invention relates to the treatment of Epidermolysis Bullosa, particularly the recessive dystrophic subtype (RDEB), using the Clustered- Regularly Interspaced Short Palindromic Repeats (CRISPR) system. This technology offers the possibility to design a single guide RNA (sgRNA) which is incorporated into a CRISPR- associated protein (Cas9) to recognize and induce DNA double-strand breaks at a specific target location. DNA double-strand breaks will be repaired by homologous recombination (HR) in the presence of a donor sequence for Epidermolysis Bullosa gene repair. In the context of Epidermolysis Bullosa, this allows to repair the mutation/s causing the disease.

### Background of the invention

Epidermolysis Bullosa is a group of rare genetic diseases characterized by strong skin fragility. The recessive dystrophic subtype, RDEB, is the most severe phenotype of the disease, causing skin and mucous blistering formation, pseudosyndactyly and a highly risk of metastatic squamous cell carcinoma development. Mutations along *COL7A1* gene, expressing collagen VII (C7), are present in a high percentage of these patients establishing this gene as target for precision medicine therapies in RDEB.

During the last years, different site-specific nucleases able to perform double strand breaks in the DNA has been explored as tools for genome editing, such as meganucleases, zinc-finger nucleases (ZFNs), transcription activator-like effector nucleases (TALENs) and CRISPR/Cas9. Genome editing-based approaches take advantage of the natural DNA repairing machinery of the cells triggered by the nuclease-induced double strand breaks (ds-breaks) to introduce INDELs in the gene sequence (NHEJ) or to precisely correct it by means of a donor template (HDR). NHEJ repairing pathway is more frequent than HR, but recent tool developments have increased the efficiencies of this donor-based correction in different cell types.

Mainly, keratinocytes and fibroblast have been highlighted as cell targets for gene therapy correction of EB. In 2013, Osborn et al. demonstrated 2% of HDR correction by using TALENs and an oligonucleotide donor (ODN) in RDEB patient-derived fibroblasts. Later, Izmyrian (2016) and collaborators developed an HDR-based correction by means of Meganucleases, achieving 4% of *COL7A1* correction. Also, **Hainzl et al.** showed genetic and functional correction in a patient-derived RDEB keratinocytes cell line using Minicircle-based CRISPR/Cas9 for HDR. Recently, Izmiryan et al. has shown precise correction of exon 2-containing mutation RDEB primary cells, achieving Indel generation frequencies close to 30% in RDEB keratinocytes and fibroblasts with integration-deficient lentivirus-guide delivery. After donor template delivery, they achieved 11% and 15.7% of corrected *COL7A1* mRNA expression without antibiotic selection in keratinocytes and fibroblasts, respectively. In other types of EB, **Benaty and colleagues** used HR to *in situ* restore LAMB3 expression in Junctional Epidermolysis Bullosa (JEB) immortalized keratinocytes by means of an adenovector carrying-Cas9/guide RNA (gRNA) tailored to the intron 2 of LAMB3 gene and an integration defective lentiviral vector bearing a promoterless quasi-complete LAMB3 cDNA flanked by homology arms.

In this field, we recently achieved high Indel efficiencies in primary RDEB keratinocytes (close to 95%) using CRISPR/Cas9 system as ribonucleoprotein complex delivered by electroporation. On the other hand, adeno-associated viruses (AAVs) have taken the lead as vectors for donor template delivery offering higher efficiencies than IDLVs, increasing HDR ratios, but without compromising biosafety. Thus, combination of AAVs with CRISPR/Cas9 could be an interesting option as genome editing tool to exploit HDR in primary keratinocytes and consequently, a gene correction strategy for RDEB primary cells.

Despite of efficient NHEJ-based approach has been recently tested by our group for exon 80-containing mutation RDEB patients, HR correction can cover a wide number of exons within the length of the designed donor, offering one therapeutic system to correct different mutated exons in *COL7A1,* enabling the benefit for a large cohort of RDEB patients.

Beyond the main cell types in the skin, allogenic bone marrow transplantation (BMT) has been taking into account during the last years as an alternative treatment for EB (Fujita 2010, Petrof et al, 2015; Kaneda et al 2015; CL Ebens et al. 2019). In fact, Ebens et al this year have shown benefit in RDEB patients after BMT with subsequent infusions of systemic MSCs with acceptable safety to the recipients. Some of these patients showed gain of primitive anchoring fibrils (AF) and increased C7 immunostaining.. Due to the possible potential of BMT in RDEB treatment, we also include in this work a proof of principle of HDR-based gene editing with the same CRISPR/Cas9 system in CB-isolated CD34+ cells and primary MSCs from three healthy donors, confirming the potential of our approach as a platform for gene correction and cell therapy.

Therefore, this invention is an evidence of an *ex vivo* efficient marker-free HR based-strategy for gene correction of different relevant cell types for RDEB treatment.

### Brief description of the figures

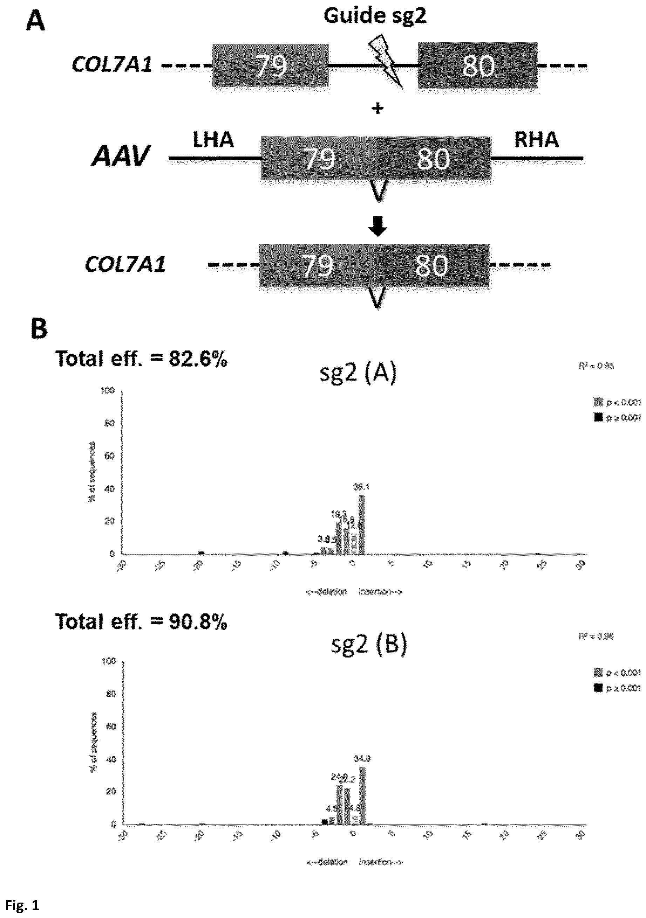

**Figure 1****.** A) Scheme of the HDR-based strategy for precise *COL7A1* correction. AAV6-delivered donor template in combination with CRISPR/Cas9 as a gene editing strategy for RDEB. CRISPR/Cas9-induced ds-breaks in the proximity of pathogenic mutations will be used to trigger HDR repair. B) TIDE analysis of indel generation within intron 79 of COL7A1. This analysis revealed highly efficient indel generation (close to 90%) in primary RDEB keratinocytes.

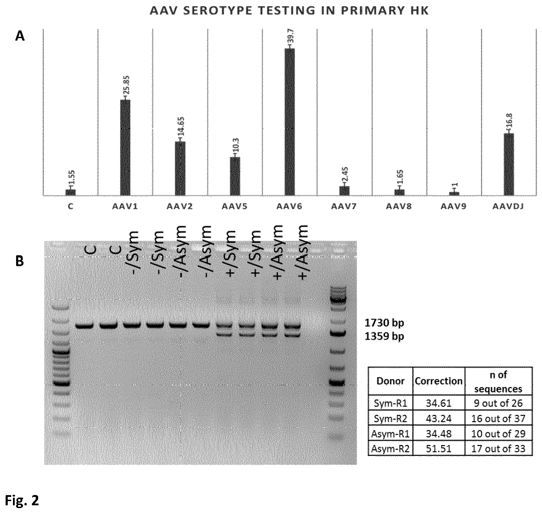

**Figure 2****.** A) AAV serotype testing in primary keratinocytes. We evaluated 8 different serotypes of AAV, showing AAV6 the highest transduction efficiency (39.7%). B) HDR-based correction genotyping in primary RDEB keratinocytes. The analysis revealed precise gene correction efficiencies close to 40% with two different donor templates tested (symmetrical and asymmetrical arms).

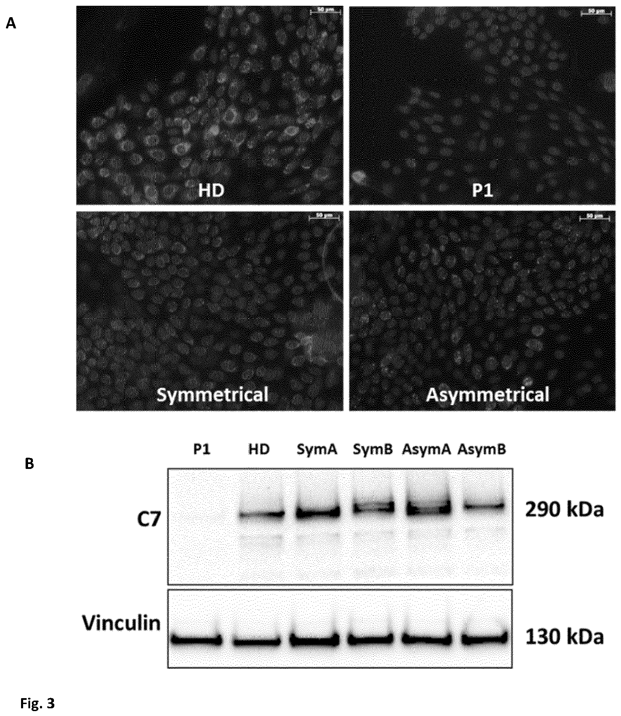

**Figure 3****.** Collagen VII expression in gene corrected RDEB polyclonal keratinocytes population. RDEB primary keratinocytes were treated with CRISPR/Cas9 and donor template containing-AAV6 and C7 expression restoration was assessed by immunofluorescense and western blot from cellular extracts. A) C7 immunofluorescense analysis. Left, upper panel: Positive control, healthy donor keratinocytes. Right, upper panel: Untreated RDEB P1 keratinocytes, showing null C7 expression. Left, lower panel: AAV6-symmetrical donor plus RNP treated RDEB keratinocytes. Right, lower panel: AAV6-asymmetrical donor template plus RNP treated RDEB keratinocytes. Bars: 50 *µ*m. B) Western blot analysis of C7 restoration in untreated, healthy and treated patient RDEB cells, showing good C7 expression consistent with the immunofluorescence images.

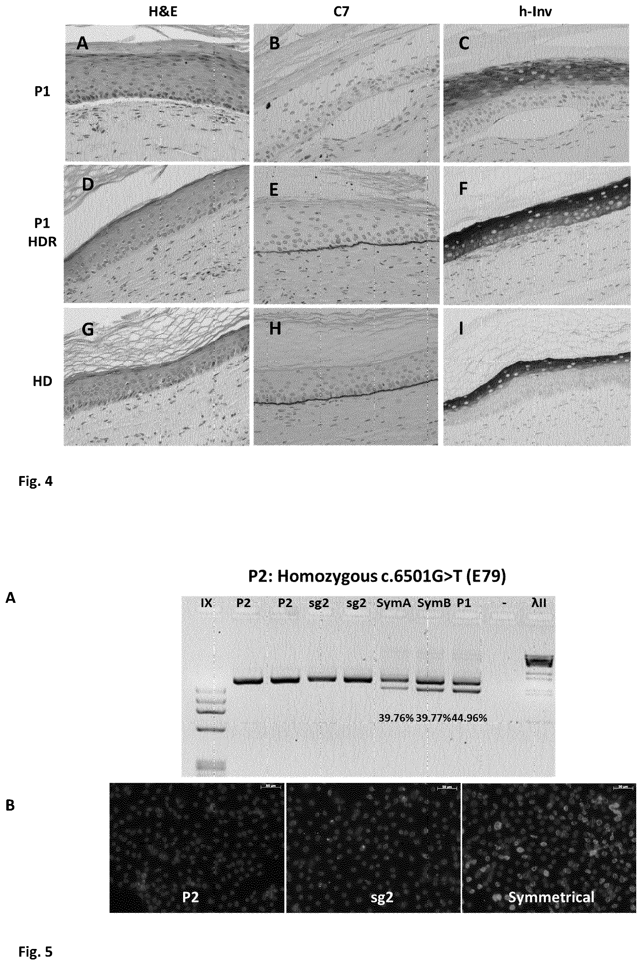

**Figure 4****.** Restoration of epidermal-dermal adhesion and C7 expression in HDR-corrected RDEB grafts. Skin equivalents containing bulk edited RDEB keratinocytes population, non-treated RDEB keratinocytes and healthy keratinocytes were transplanted onto nude mice. Histological analysis (H&E staining) of grafts (Fig. 4A, D, G) show epidermal detachment in P1 grafts. C7 expression analysis showing continuous C7 deposition at the BMZ in HDR-corrected (P1 HDR) and healthy donor (HD) keratinocytes, and no C7 detection in graft from non-treated RDEB keratinocytes (P1) (Fig. 4B, E, H). Human involucrin (h-Inv) assessment showing normal epidermal differentiation in all the grafts shown (Fig. 4C, F, I).
**Figure 5****.** A) PCR genotyping of P2 RDEB treated cells with RNP plus Donor template containing-AAV6. Similar ratios of gene correction were observed between the two RDEB treated patients. B) Immunofluorescence for C7 expression detection. P2 was null for C7 expression. After treatment, C7 expression is restored in a significant amount of cells.

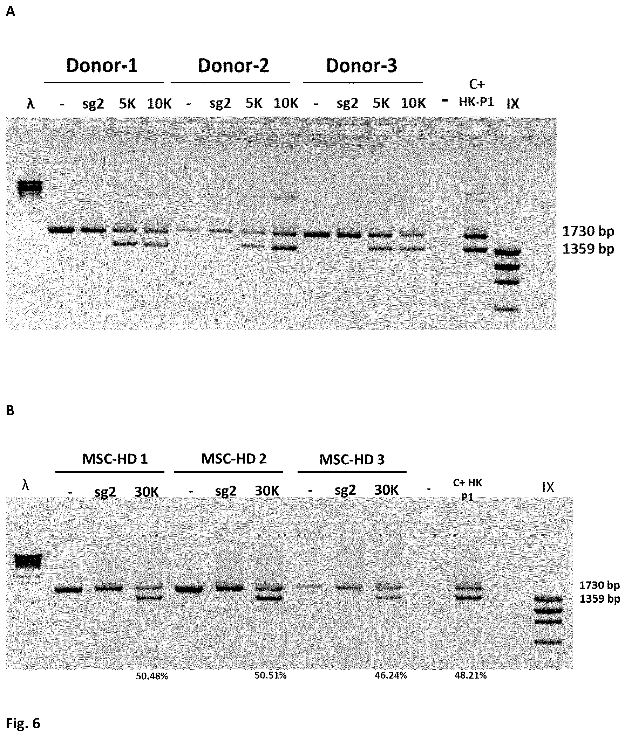

**Figure 6****.** A) HDR-based gene editing in CD34+ cells from three healthy donors. B) HDR-based gene editing in MSC in three healthy donors.

### Description of the invention

### Definitions

As used herein, the following terms have the meanings ascribed to them unless specified otherwise.

The terms "a," "an," or "the" as used herein not only include aspects with one member, but also include aspects with more than one member. For instance, the singular forms "a," "an," and "the" include plural referents unless the context clearly dictates otherwise. Thus, for example, reference to "a cell" includes a plurality of such cells and reference to "the agent" includes reference to one or more agents known to those skilled in the art, and so forth.

The term "gene" refers to a combination of polynucleotide elements, that when operatively linked in either a native or recombinant manner, provide some product or function. The term "gene" is to be interpreted broadly, and can encompass mRNA, cDNA, cRNA and genomic DNA forms of a gene.

The term "homology-directed repair" or "HDR" refers to a mechanism in cells to accurately and precisely repair double-strand DNA breaks using a homologous template to guide repair. The mechanism underlying HDR is homologous recombination (HR).

The term "homologous recombination" or "HR" refers to a genetic process in which nucleotide sequences are exchanged between two similar molecules of DNA. Homologous recombination (HR) is used by cells to accurately repair harmful breaks that occur on both strands of DNA, known as double-strand breaks or other breaks that generate overhanging sequences.

The term "single guide RNA" or "sgRNA" refer to a DNA-targeting RNA containing a guide sequence that targets the Cas nuclease to the target genomic DNA and a scaffold sequence that interacts with the Cas nuclease (e.g., tracrRNA).,

The term "Cas polypeptide" or "Cas nuclease" refers to a Clustered Regularly Interspaced Short Palindromic Repeats-associated polypeptide or nuclease that cleaves DNA to generate blunt ends at the double-strand break at sites specified by a 20-nucleotide guide sequence contained within the crRNA molecule. A Cas nuclease requires both a crRNA and a tracrRNA for site-specific DNA recognition and cleavage. The crRNA associates, through a region of partial complementarity, with the tracrRNA to guide the Cas nuclease to a region homologous to the crRNA in the target DNA called a "protospacer."

The term "ribonucleoprotein complex" or "RNP complex" refers to a complex comprising an sgRNA and a Cas polypeptide.

The term "Adeno associated viral vector-delivered donor template" or "donor template-containing adeno-associated viral vector" refers to an adeno-associated viral particle that can deliver a recombinant donor template for CRISPR-based gene editing via homology-directed repair in a target cell, e.g., primary cell.

The term "recombinant donor template" refers to a nucleic acid strand, e.g., DNA strand that is the donor strand during homologous recombination strand invasion that is initiated by the damaged DNA repair mechanism, in some cases, resulting from a double-stranded break. The donor polynucleotide serves as template material to direct the repair of the damaged DNA region.

The terms "sequence identity" or "percent identity" in the context of two or more nucleic acids or polypeptides refer to two or more sequences or subsequences that are the same ("identical") or have a specified percentage of amino acid residues or nucleotides that are identical ("percent identity") when compared and aligned for maximum correspondence with a second molecule, as measured using a sequence comparison algorithm (e.g., by a BLAST alignment, or any other algorithm known to persons of skill), or alternatively, by visual inspection.

The term "homologous" refers to two or more amino acid sequences when they are derived, naturally or artificially, from a common ancestral protein or amino acid sequence. Similarly, nucleotide sequences are homologous when they are derived, naturally or artificially, from a common ancestral nucleic acid.

The term "primary cell" refers to a cell isolated directly from a multicellular organism. Primary cells typically have undergone very few population doublings and are therefore more representative of the main functional component of the tissue from which they are derived in comparison to continuous (tumor or artificially immortalized) cell lines. In some cases, primary cells are cells that have been isolated and then used immediately. In other cases, primary cells cannot divide indefinitely and thus cannot be cultured for long periods of time in vitro.

The term "gene modified primary cell" or "genome edited primary cell" refers to a primary cell into which a heterologous nucleic acid has been introduced in some cases, into its endogenous genomic DNA.

The term "pharmaceutical composition" refers to a composition that is physiologically acceptable and pharmacologically acceptable. In some instances, the composition includes an agent for buffering and preservation in storage, and can include buffers and carriers for appropriate delivery, depending on the route of administration.

The term "pharmaceutical acceptable carrier" refers to a substance that aids the administration of an agent (e.g., Cas nuclease, modified single guide RNA, gene modified primary cell, etc.) to a cell, an organism, or a subject. "Pharmaceutically acceptable carrier" refers to a carrier or excipient that can be included in a composition or formulation and that causes no significant adverse toxicological effect on the patient. Non-limiting examples of pharmaceutically acceptable carrier include water, NaCl, normal saline solutions, lactated Ringer's, normal sucrose, normal glucose, binders, fillers, disintegrants, lubricants, coatings, sweeteners, flavors and colors, and the like. One of skill in the art will recognize that other pharmaceutical carriers are useful in the present invention.

The term "administering or "administration" refers to the process by which agents, compositions, dosage forms and/or combinations disclosed herein are delivered to a subject for treatment or prophylactic purposes. Compositions, dosage forms and/or combinations disclosed herein are administered in accordance with good medical practices taking into account the subject's clinical condition, the site and method of administration, dosage, subject age, sex, body weight, and other factors known to the physician. For example, the terms "administering" or "administration" include providing, giving, dosing and/or prescribing agents, compositions, dosage forms and/or combinations disclosed herein by a clinician or other clinical professional.

The term "treating" refers to an approach for obtaining beneficial or desired results including but not limited to a therapeutic benefit and/or a prophylactic benefit. By therapeutic benefit is meant any therapeutically relevant improvement in or effect on one or more diseases, conditions, or symptoms under treatment. For prophylactic benefit, the compositions may be administered to a subject at risk of developing a particular disease, condition, or symptom, or to a subject reporting one or more of the physiological symptoms of a disease, even though the disease, condition, or symptom may not have yet been manifested.

The terms "subject," "patient," and "individual" are used herein interchangeably to include a human or animal. For example, the animal subject may be a mammal, a primate (e.g., a monkey), a livestock animal (e.g., a horse, a cow, a sheep, a pig, or a goat), a companion animal (e.g., a dog, a cat), a laboratory test animal (e.g., a mouse, a rat, a guinea pig, a bird), an animal of veterinary significance, or an animal of economic significance.

Unless defined otherwise, all technical and scientific terms used herein have the same meanings as commonly understood by one of ordinary skill in the art to which this technology belongs. Although exemplary methods, devices and materials are described herein, any methods and materials similar or equivalent to those expressly described herein can be used in the practice or testing of the present technology. For example, the reagents described herein are merely exemplary and that equivalents of such are known in the art. The practice of the present technology can employ, unless otherwise indicated, conventional techniques of tissue culture, immunology, molecular biology, microbiology, cell biology, and recombinant DNA, which are within the skill of the art. See, e.g., Sambrook and Russell eds. (2001) Molecular Cloning: A Laboratory Manual, 3rd edition; the series Ausubel et al. eds. (2007) Current Protocols in Molecular Biology; the series Methods in Enzymology (Academic Press, Inc., N.Y.); MacPherson et al. (1991) PCR I: A Practical Approach (IRL Press at Oxford University Press); MacPherson et al. (1995) PCR 2: A Practical Approach; Harlow and Lane eds. (1999) Antibodies, A Laboratory Manual; Freshney (2005) Culture of Animal Cells: A Manual of Basic Technique, 5th edition; Miller and Calos eds. (1987) Gene Transfer Vectors for Mammalian Cells (Cold Spring Harbor Laboratory); and Makrides ed. (2003) Gene Transfer and Expression in Mammalian Cells (Cold Spring Harbor Laboratory).

### Description

Genetically modified stem cells are offering a new field of therapeutic solutions for untreatable diseases. In hematology, CD34+ gene corrected cells have shown a great benefit for the treatment of severe blood disorders. Moreover, the newest gene modification technologies have reached clinical trials stage (CRISPR) making a revolution in modern medicine. Also, MSCs therapy is showing benefits at clinical stage for wound healing and immunological disorders treatment, offering a safe approach for regenerative medicine.

In skin disorders, **keratinocytes and fibroblasts** are a cell source for these therapies and so many approaches are being developed aiming to pave the way for clinical translation. Epidermolysis bullosa is one of the most devastating skin rare diseases and RDEB-subtype, with complete absence of C7 expression, is considered the most severe subtype. A large number of mutations in these patients have been described within COL7A1 gene, making this gene the main target of gene therapy strategies to correct RDEB. Recently, benefits have been shown in an *ex vivo* phase I clinical trial in patients transplanted with **skin equivalents** containing autologous epidermal stem treated with gamma-retrovirus expressing cDNA C7 sequence, as a classical gene therapy approach. Patients showed wound healing amelioration and C7 deposition and anchoring fibrils formation.

But, new gene editing tools are opening the way for more precise gene correction therapies. In fact, we have previously shown a highly efficient gene editing-based approach for E80 correction in RDEB patient cells. This work revealed CRISPR/Cas9 as RNP as the most efficient tool for genome editing in primary keratinocytes, a cell type considered hard-to-transfect. In the present invention, we have tested, for the first time, a large collection of AAV serotypes to find the highest transduction efficiency as possible, and we found that, after electroporation, the AAV6 serotype provided the best performance. Thus, we have shown that a donor template containing-AAV6 plus RNP delivered by electroporation is an efficient tool for genome editing in keratinocytes.

Testing different homology donor designs, we found similar HR-based correction ratios in primary keratinocytes, independent of the grade of symmetry in the homology arms respect to the cutting site. In our study, symmetrical donor covers from E74 to E84 and asymmetrical donor template from E77 to E88, so each design could cover patients with mutations within 10 different exons of COL7A1. Even more, we could develop a large collection of AAVs containing different gene regions of COL7A1, able to correct any mutation along the gene. This is an important advantage against the previous NHEJ proposed because there are some exons that are not amenable to exon removal, like exon 1, 2, 3, 24, 27 or 113, among others, that could be corrected by precise HR-mediated gene correction. Beyond EB treatment, this approach could be transfer to any genome editing application in primary keratinocytes, to treat mutations from other skin diseases or even knock-in genes (reporters, therapeutic molecules) and create skin with new functional properties.

Previous works have shown HR-based gene correction in different cell types relevant to EB treatment. In fact, previous works have demonstrated the feasibility to achieve HR-correction in a patient-derived keratinocytes cell line by means of TALENs and selection cassette containing-AAV. After selection treatment on treated keratinocytes, 32 out of 34 clones isolated were genetically corrected. But, although it demonstrated the feasibility of C7 restoration by HR in a RDEB keratinocyte cell line, drug selection and clone isolation make very difficult the translation into clinic of this type of therapies. However, others have demonstrated in exon 2-containing mutation RDEB keratinocytes indel generation frequencies close to 30% in RDEB keratinocytes by means of integration-deficient lentivirus-guide delivery, that in combination with a donor template delivery, achieved 11% and 15% of COL7A1 corrected transcripts in RDEB cells, keratinocytes and fibroblasts respectively, and up to 19% in skin grafts was enough to allow AF formation with no dermal-epidermal separation.

For the present invention, we provide a marker-free highly efficient approach in primary keratinocytes capable of **achieving close to 40% of corrected transcripts in primary keratinocytes, surpassing previous HR-based gene correction ratios.** In addition, we have produced an edited bulk keratinocyte population capable of producing normal human skin regeneration with restoration of the dermo-epidermal adhesion when transplanted onto nude mice. Using a polyclonal population makes easier the translation into clinics, avoiding time-consuming and laborious epidermal clone isolation.

| **Study** | **Genome editing tool** | **% of HDR correction** |
|---|---|---|
| **Osborn et al, 2013** | TALENs | 2% |
| **Izmyrian et al, 2016** | Meganucleases | 4% |
| **Hainzl et al, 2017** | Minicircle-based CRISPR/Cas9 | 17-24% (after marker selection) |
| **Izmyrian et al, 2018** | Integration-deficient lentivirus-guide delivery (CRISPR/Cas9) | 11% and 15.7% |

It is believed that 35% of normal C7 levels are necessary for skin mechanical stability based on wild-type (WT) fibroblast injection experiments in a DEB hypomorphic murine model. Reference: Georgiadis, C., Syed, F., Petrova, A., Abdul-Wahab, A., Lwin, S.M., Farzaneh, F., Chan, L., Ghani, S., Fleck, R.A., Glover, L., et al. (2016). Lentiviral Engineered Fibroblasts Expressing Codon-Optimized COL7A1 Restore Anchoring Fibrils in RDEB. J. Invest. Dermatol. 136, 284-292.).

Therefore, in contrast to other methods, the methodology presented herein surpasses the percentage of corrected transcripts in primary keratinocytes needed to adequately treat or prevent EB. On the whole, our Invention offers evidence of an *ex vivo* effective genome editing tool able to achieve gene correction in many different cell types, providing a source to develop different cell therapies aiming EB cure. A wider spectrum of EB mutations is covered by this technology, paving the way for clinical benefit of a large cohort of EB patients.

Therefore, in a first aspect of the invention, we herein provide a method for inducing a stable gene modification of a target nucleic acid comprising one or more Epidermolysis Bullosa, preferably recessive Dystrophic Epidermolysis Bullosa (RDEB), disease-causing mutations of the COL7A1 gene via homologous recombination in a primary cell, preferably in primary keratinocytes, fibroblasts or skin stem cells. The method includes introducing into the primary cell: (a) a modified single guide RNA (sgRNA) comprising a nucleotide sequence that is complementary to the target nucleic acid and a nucleotide sequence that interacts with a CRISPR-associated protein (Cas) polypeptide, wherein the RNA component can be two individual RNA molecules (crRNA and tracrRNA) or a single RNA molecule (sgRNA); (b) a Cas polypeptide, an mRNA encoding a Cas polypeptide, and/or a recombinant expression vector comprising a nucleotide sequence encoding a Cas polypeptide, wherein the modified sgRNA, or crRNA and tracrRNA components provided separately, guide the Cas polypeptide to the target genomic sequence to be corrected; and (c) a homologous donor, preferably wild/type, adeno-associated viral serotype 6 (AAV-6) or 1 (AAV-1) comprising a recombinant donor template comprising two nucleotide sequences comprising two non-overlapping, homologous portions of the target nucleic acid, wherein the nucleotide sequences are located at the 5' and 3' ends of a nucleotide sequence corresponding to the target nucleic acid to undergo homologous recombination; wherein, the stable gene modification of the target nucleic acid comprises the replacement of disease-causing mutations of the COL7A1 gene (target nucleic acid), preferably in any of exons 73, 74, 75, 80 or 105 of the COL7A1 gene, preferably those disease-causing mutations with a high frequency of heterozygosity in the human population, by introducing the homologous donor AAV-6 or AAV-1 vector comprising the correction donor template.

The above-mentioned gene modification strategy in a primary cell, preferably in primary keratinocytes, is preferably performed with the aim of treating a subject having or suffering from Epidermolysis Bullosa, preferably from recessive Dystrophic Epidermolysis Bullosa (RDEB). It is noted that recessive Dystrophic Epidermolysis Bullosa (RDEB) is an inherited genetic blistering skin disorder caused by mutations in the COL7A1 gene (collagen VII, C7) leading to lack of C7 function. Type VII collagen, (C7) is a large homotrimeric triple helical collagenous molecule, which undergoes anti-parallel dimer formation at its NC2 end, followed by supramolecular assembly into attachment structures termed anchoring fibrils, which connect the lamina densa of the BMZ to the papillary dermis. C7 contains a large NC1 domain, which binds laminin-332 in the lamina densa and a collagenous domain, which wraps around interstitial collagen fibrils in the papillary dermis. Thus, lack of C7 in RDEB produces blistering between the papillary dermis and lamina densa. The human type VII collagen gene, COL7A1 has a complex structure consisting of a total of 118 separate exons. The gene is, however, relatively compact, and most of the introns are relatively small; consequently, the size of the entire human COL7A1 gene is only -32 kb, encoding a messenger RNA of -8.9 kb. COL7A1 has been mapped to the short-arm of human chromosome 3, region 3p21.1. The type VII collagen gene structure and the encoded primary sequence of the protein are well conserved, and for example, the mouse gene shows 84.7 percent homology at the nucleotide and 90.4 percent identity at the protein level.

Type VII collagen is synthesized both by epidermal keratinocytes and dermal fibroblasts in culture. Upon synthesis of complete pro-al (VII) polypeptides, three polypeptides associate through their carboxy-terminal ends to a trimer molecule which in its collagenous portion folds into the triple-helical formation. The triple-helical molecules are then secreted to the extracellular milieu where two type VII collagen molecules align into an anti-parallel dimer with the amino-terminal domains present at both ends of the molecule. This dimer assembly is accompanied by proteolytic removal of a portion of the carboxy-terminal end of both type VII collagen molecules and stabilization by inter-molecular disulfide bond formation. Subsequently, a large number of these anti-parallel dimers aggregate laterally to form anchoring fibrils.

Glycine substitution mutations in the triple helical domain of COL7A (especially in exons 73, 74, and 75) predominate in dominant dystrophic epidermolysis bullosa (DDEB). Mutations p.Gly2034Arg and p.Gly2043Arg are the most common DDEB-causing mutations, making up 50% of the dominant mutations reported in the largest US cohort. Glycine substitutions as well as other amino acid substitutions and splice junction mutations outside of this region may also be found in dominant DEB. More than 400 recessive DEB-causing mutations spanning the entire gene have been described for all forms of DEB. Each mutation, however, accounts for no more than 1%-2% of the total number of mutations described. Null mutations predominate in RDEB, though glycine substitutions and other amino acid substitutions have been described. Milder forms of RDEB are often caused by splice junction mutations or other missense mutations.

Therefore, in further embodiments, the stable gene modification of the target nucleic acid comprises the replacement of any of the above-mentioned disease-causing mutations of the COL7A1 gene by introducing a homologous donor AAV-6 or AAV-1 vector comprising correction donor template.

In some embodiments, the primary cell is selected from the group consisting of a primary keratinocyte or a fibroblast, and a combination thereof. In some embodiments, the primary cell is isolated from a mammal prior to introducing the modified sgRNA, the Cas polypeptide, and the homologous donor AAV vector into the primary cell. For instance, the primary cell can be harvested from a human subject. In some instances, the primary cell or a progeny thereof is returned to the mammal after introducing the modified sgRNA, the Cas polypeptide, and the homologous donor AAV vector into the primary cell. In other words, the genetically modified primary cell undergoes autologous transplantation. In other instances, the genetically modified primary cell undergoes allogeneic transplantation. For example, a primary cell that has not undergone stable gene modification is isolated from a donor subject, and then the genetically modified primary cell is transplanted into a recipient subject who is different than the donor subject.

The primary cell can comprise a population of primary cells. In some cases, the population of primary cells comprises a heterogeneous population of primary cells. In other cases, the population of primary cells comprises a homogeneous population of primary cells.

In some instances, the homologous donor AAV-6 vector has at least about 90% sequence identity to AAV6. In other instances, the homologous donor is a wild-type AAV6 or an AAV6 variant having at least 95% sequence identity to wild-type AAV6, e.g., 95%, 96%, 97%, 98%, 99%, or 100% sequence identity to wild-type AAV6. In some embodiments, polynucleotides encoding one or more of the various components of the AAV-6 vector are operably linked to an inducible promoter, a repressible promoter, or a constitutive promoter. In addition, regulatory sequences operably linked to the components can include activator binding sequences, enhancers, introns, polyadenylation recognition sequences, promoters, repressor binding sequences, stem-loop structures, translational initiation sequences, translation leader sequences, transcription termination sequences, translation termination sequences, primer binding sites, and the like. Commonly used promoters are constitutive mammalian promoters CMV, EFIa, SV40, PGKI (mouse or human), Ubc, CAG, CaMKIIa, and beta-Act, and others known in the art (Khan, K. H. (2013) "Gene Expression in Mammalian Cells and its Applications," Advanced Pharmaceutical Bulletin 3(2), 257- 263). Further, mammalian RNA polymerase III promoters, including HI and U6, can be used.

In some embodiments, a recombinant mammalian expression vector is capable of preferentially directing expression of the nucleic acid in a particular cell type (e.g., using tissue-specific regulatory elements to express a polynucleotide). Tissue-specific regulatory elements are known in the art and include, but are not limited to, the albumin promoter, lymphoid-specific promoters, neuron-specific promoters (e.g., the neurofilament promoter), pancreas-specific promoters, mammary gland-specific promoters (e.g., milk whey promoter), and in particular promoters of T cell receptors and immunoglobulins. Developmentally- regulated promoters are also encompassed, e.g., the murine hox promoters and the alpha- fetoprotein promoter.

Methods of introducing the AAV-6 or AAV-1 expression vector into host cells are known in the art and are typically selected based on the kind of host cell.

In some embodiments, the stable gene modification of the target nucleic acid is induced in greater than about 30% of the population of primary cells, e.g., about 35%, about 40%, about 50%, about 60%, about 70% about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 100% of the population of primary cells. In other embodiments, the stable gene modification of the target nucleic acid is induced in greater than about 80% of the population of primary cells, e.g., about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 100% of the population of primary cells. In yet other embodiments, the stable gene modification of the target nucleic acid is induced in greater than about 90% of the population of primary cells, e.g., about 90%, about 91%, about 92%, about 93%, about 94%, about 95%, about 96%, about 97%, about 98%, about 99%, or about 100% of the population of primary cells.

In some embodiments, the sequences of the first aspect of the invention may comprise modified nucleotides such as a modification in a ribose group, a phosphate group, a nucleobase, or a combination thereof. In some instances, the modification in the ribose group comprises a modification at the 2' position of the ribose group. In some cases, the modification at the 2' position of the ribose group is selected from the group consisting of 2'-O-methyl, 2'-fluoro, 2'-deoxy, 2'-O-(2-methoxyethyl), and a combination thereof. In other instances, the modification in the phosphate group comprises a phosphorothioate modification. In other embodiments, the modified nucleotides are selected from the group consisting of a 2'-O-methyl (M) nucleotide, a 2'-O-methyl 3'-phosphorothioate (MS) nucleotide, a 2'-O-methyl 3'-thioPACE (MSP) nucleotide, and a combination thereof.

In some embodiments, the Cas polypeptide is a Cas9 polypeptide, a variant thereof, or a fragment thereof. In certain instances, the Cas polypeptide variant comprises a high-fidelity or enhanced specificity Cas9 polypeptide variant. In certain embodiments, the modified sgRNA and the Cas polypeptide are introduced into the primary cell concomitantly. In other embodiments, the modified sgRNA and the Cas polypeptide are introduced into the primary cell sequentially. In some cases, the modified sgRNA is introduced first, and the Cas polypeptide thereafter. In other cases, the Cas polypeptide is introduced first, and the modified sgRNA thereafter.

In some embodiments, the modified sgRNA and the Cas polypeptide can be incubated together to form a ribonucleoprotein (RNP) complex prior to introducing into the primary cell. For instance, the modified sgRNA and the Cas polypeptide can be mixed together in a vessel to form an RNP complex, and then the RNP complex is introduced into the primary cell. In other embodiments, the Cas polypeptide described herein can be an mRNA encoding the Cas polypeptide, which Cas mRNA is introduced into the primary cell together with the modified sgRNA as an "All RNA" CRISPR system. In certain instances, the modified sgRNA and the Cas mRNA are introduced into the primary cell concomitantly. In other instances, the modified sgRNA and the Cas mRNA are introduced into the primary cell sequentially. In some cases, the modified sgRNA is introduced first, and the Cas mRNA thereafter. In other cases, the Cas mRNA is introduced first, and the modified sgRNA thereafter.

In some embodiments, the RNP complex and the homologous donor AAV-6 or AAV-1 vector are concomitantly introduced into the primary cell. In other embodiments, the RNP complex and the homologous donor AAV-6 vector are sequentially introduced into the primary cell. In some instances, the RNP complex is introduced into the primary cell before the homologous donor AAV vector. In other instances, the homologous donor AAV vector is introduced into the primary cell before the RNP complex. For example, the RNP complex can be introduced into the primary cell about 1, 2, 3, 4, 5, 6, 7, 8, 9, 10, 11, 12, 13, 14, 15, 16, 17, 18, 19, 20, 25, 30, 35, 40, 45, 50, 55, 60, 90, 120, 150, 180, 210, or 240 minutes or more before the homologous donor AAV vector, or vice versa. In particular embodiments, the RNP complex is introduced into the primary cell about 15 minutes (e.g., from about 10 to about 20 minutes) before the homologous donor AAV-6 vector.

In some embodiments, the "All RNA" CRISPR system and the homologous donor AAV vector are concomitantly introduced into the primary cell. In other embodiments, the "All RNA" CRISPR system and the homologous donor AAV-6 vector are sequentially introduced into the primary cell. In some instances, the "All RNA" CRISPR system is introduced into the primary cell before the homologous donor AAV-6 vector. In other instances, the homologous donor AAV-6 vector is introduced into the primary cell before the "All RNA" CRISPR system. For example, the "All RNA" CRISPR system can be introduced into the primary cell about 1, 2, 3, 4, 5, 6, 7, 8, 9, 10, 11, 12, 13, 14, 15,16,17, 18,19, 20, 25, 30, 35,40, 45, 50, 55, 60, 90,120, 150, 180, 210, or 240 minutes or more before the homologous donor AAV vector, or vice versa. In particular embodiments, the "All RNA" CRISPR system is introduced into the primary cell about 15 minutes (e.g., from about 10 to about 20 minutes) before the homologous donor AAV vector.

In some embodiments, any of the methods described herein can also include purifying the primary cell having the stable gene modification of the target nucleic acid using the marker. In some cases, the composition isolated by the purifying step includes at least about 80% primary cells having the stable gene modification of the target nucleic acid, e.g., about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or more primary cells having the stable gene modification of the target nucleic acid.

In some embodiments, the step of introducing the modified sgRNA and the Cas polypeptide into the primary cell comprises electroporating the modified sgRNA and the Cas polypeptide into the primary cell. In some embodiments, the step of introducing the homologous donor AAV-6 or AAV-1 vector into the primary cell comprises transducing the primary cell.

In other aspects, provided herein is a genetically modified primary cell produced by any of the methods described herein. In some embodiments, the genetically modified primary cell is selected from the group consisting of a primary keratinocyte or a fibroblast, and a combination thereof.

In yet other aspects, provided herein is a pharmaceutical composition comprising any of the genetically modified primary cells described herein, and a pharmaceutically acceptable carrier. In other embodiments, the pharmaceutical composition comprises one type of genetically modified primary cell. In other embodiments, the pharmaceutical composition comprises two or more different types of genetically modified primary cells, e.g., 2, 3, 4, 5, 6, 7, 8, 9, 10 or more different types of genetically modified primary cells.

In further aspects, provided herein is the *in vitro* use of a kit comprising (a) a single guide RNA (sgRNA) comprising a first nucleotide sequence that is complementary to the target nucleic acid and a second nucleotide sequence that interacts with a CRISPR-associated protein (Cas) polypeptide; (b) a Cas polypeptide, an mRNA encoding a Cas polypeptide, and/or a recombinant expression vector comprising a nucleotide sequence encoding a Cas polypeptide, wherein the sgRNA guides the Cas polypeptide to the target nucleic acid; (c) a homologous donor adeno-associated viral (AAV6) or AAV-1 vector comprising a recombinant donor template comprising two nucleotide sequences comprising two non-overlapping, homologous portions of the target nucleic acid, wherein the nucleotide sequences are located at the 5' and 3' ends of a nucleotide sequence corresponding to the target nucleic acid to undergo homologous recombination, and optionally an instruction manual, in a method for inducing a stable gene modification of a target nucleic acid comprising one or more Epidermolysis Bullosa, preferably recessive Dystrophic Epidermolysis Bullosa (RDEB), disease-causing mutations of the COL7A1 gene via homologous recombination in a primary cell, preferably in primary keratinocytes or fibroblasts obtained from a subject.

In some instances, the kit also contains a reagent for harvesting or isolating a primary cell from a subject. The subject can be a mammalian subject, e.g., a human subject.

In yet further aspects, provided herein is method of preventing or treating Epidermolysis Bullosa, preferably recessive Dystrophic Epidermolysis Bullosa (RDEB), in a subject in need thereof, the method comprising administering to the subject any of the genetically modified primary cells described herein, or any of the pharmaceutical compositions described herein, to prevent the disease or ameliorate one or more symptoms of the disease.

In some embodiments, the step of administering comprises a delivery route selected from the group consisting of intravenous, intraperitoneal, intramuscular, intradermal, subcutaneous, intrathecal, intraosseous, or a combination thereof.

In particular embodiments, the genetically modified primary cells or pharmaceutical compositions of the present invention are administered to the subject in a sufficient amount to correct a mutation in the target nucleic acid that is associated with the disease. In some instances, the mutation is corrected by replacing a mutant allele in the target nucleic acid with the wild-type allele.

In further embodiments of the present invention, the genetically modified primary cells or pharmaceutical compositions of the present invention are use in *vitro* to manufacture skin equivalents or artificial skin. Still further embodiments of the present invention are thus directed to skin equivalents obtainable or obtained according to the previously mentioned in vitro use. Still further embodiments are directed to such skin equivalents obtainable or obtained according to the previously mentioned in vitro use, for use in a method of treatment of Epidermolysis Bullosa, particularly the recessive dystrophic subtype (RDEB) in a subject in need thereof.

Other objects and advantages of the invention will become apparent to those skilled in the art from a review of the ensuing detailed description, which proceeds with reference to the following illustrative drawings, and the attendant claims.

Consequently, the applicants have herein shown the use of the CRISPR system to definitely repair the RDEB mutation. Applicants target sites around the mutated site. DNA repair of RDEB's disease mutation using the CRISPR/Cas9 system represents a new and original therapeutic approach. The present invention offers the possibility to act at the DNA level with engineered nucleases to inactivate or repair a disease-causing mutation.

The following examples are merely illustrative and do not limit the scope of the present invention.

### Examples

### Materials and methods

### Keratinocytes cell culture and isolation of clones

Patient keratinocytes were originally obtained from skin biopsies of three RDEB (RDEB-sev gen) patients carrying mutations in the *COL7A1* gene. Skin biopsies were obtained from patients after approval from the Ethics Committee of the collaborating hospital upon informed consent.

Primary human RDEB and healthy donor keratinocytes were cultured. Human primary RDEB keratinocytes from the three patients were plated onto lethally irradiated 3T3-J2 cells and cultured in a keratinocyte growth cFAD medium (KCa), a 3:1 mix of Dulbecco's modified Eagle's and Ham's F12 media (GIBCO-BRL, Barcelona, Spain) containing fetal bovine calf serum (Hyclone, GE Healthcare, Logan, UT) (10%), penicillin-streptomycin (1%), glutamine (2%), insulin (5 µg/ml; Sigma Aldrich), adenine (0.18 mmol/l; Sigma Aldrich), hydrocortisone (0.4 µg/ml; Sigma Aldrich), cholera toxin (0.1 nmol/l; Sigma Aldrich), triiodothyronine (2 nmol/l; Sigma Aldrich), EGF (10 ng/ml; Sigma Aldrich) and Y-27632 ROCK inhibitor (Sigma Aldrich) at 10µM. To obtain isolated clones, cells were then trypsinized and plated at low density in 100 mm plates (10³cells/plate) with 2x10⁶ lethally irradiated 3T3 feeder cells per plate. Cell clones were then collected using polystyrene cloning cylinders (Sigma, St Louis, MO) and expanded for further analysis.

### Donor containing-AAV6 production

Homology arms were amplified by PCR from wild-type genomic DNA. The symmetric donor is a fusion of exon 79 and exon 80, missing intron 79, where sg2 cuts. So, left homology arm (LHA) is 1008 bp from the end of exon 79 to 5' of COL7A1 gene and right homology arm (RHA) is 798 bp from the beginning of exon 80 to 3' of this gene. Asymmetric donor follows same strategy, but LHA is 556 bp and RHA 1461 bp. Then, both arms were assembled with an AAV backbone plasmid by Gibson assembly technology.

For donor template containing-AAV6 production, backbone vector plasmids were grown in E.coli and isolated by means of Endotoxin-Free Maxi Plasmid Purification Kit (Invitrogen, Cat# A33073). Then, five 293 cells 15cm² dishes were transfected using 120uL 1 mg/mL PEI per plate (MW 25K)(Polysciences) mixed with 6 mg ITR-containing plasmid and 22 mg pDGM6 (which carried AAV6 cap, AAV2 rep, and adenoviral helper genes)(gift from D. Russell). 72h after transfection, vectors were purified using a Takara AAVpro Purification Kit (Cat. 6666), following manufacturer's protocol. Vector titer was assessed by ddPCR using probes on the ITRs region.

### CRISPR/Cas9 delivery and AAV6 transduction

Sg2 gRNA was previously described (Bonafont et al.). In this approach, instead of crRNA:tracrRNA system, sg2 was a single guide RNA and chemically modified (Synthego, CA, USA). 1.6 ug of sgRNA mixed with 6 ug of Cas9 protein were delivered by electroporation in each reaction for 1x10⁵ primary keratinocytes (Integrated DNA Technologies, IA, USA). Electroporation platform used for the delivery of the RNP was 4D-Nucleofector™ System (Lonza Bioscience, Switzerland), electroporation code CM137.

After electroporation, cells were transduced in suspension for 1 hour with the donor containing-AAV6 (MOI 30K) in a final volume of 50 ul with Opti-MEM (ThermoFisher Scientific). Then, cells were plated onto feeder layer-containing plates.

For MSC and CD34⁺ cells, 3.2 ug of sgRNA and 6 ug of Cas9 were used. The electroporation code used for MSC electroporation was CM119 and DZ100 was the one used for CD34⁺ cells transfection. For transduction, MSC were incubated with the AAV6 for 15 minutes in suspension and then they were plated on media. In the case of CD34+ cells, AAV6 was added directly to the well.

### Genotyping of gene- targeted keratinocytes

6 days post-treatment, genomic DNA was isolated by isopropanol precipitation of keratinocyte lysates (lysis buffer was Tris pH8 100 mM, EDTA 5 mM, SDS 0.2%, NaCl 200 mM, 1mg/ml proteinase K (Roche Diagnostics, Mannheim, Germany) and resuspended in TE buffer. Approximately 50 ng of genomic DNA were used for PCR amplification. PCR fragments spanning the target region were generated with primers S1 F/R, outside the homology arms. F: 5'-CACCAGCATTCTCTCTTCC-3'; R: 5'- GTTCTT GGG TAC TCACCA C-3'. PCR program was: 98°C for 1 minute, 5 cycles of 98°C for 30 seconds, 68°C for 30 seconds, 72°C for 45 seconds, decreasing annealing temperature 1°C every cycle, followed by 30 cycles of 94°C for 30 seconds, 63°C for 30 seconds, 72°C for 45 seconds, then 72°C for 10 minutes. PCR products were analyzed in 1.5% agarose gel. Molecular weight marker was IX (Sigma-Aldrich). For sequencing, PCR products were treated with illustra™ ExoProStar™(GE Healthcare, UK), sequenced using Big Dye Terminator V.1.1 Cycle Sequencing kit (Thermo Fisher, Waltham, MA), and examined on a 3730 DNA Analyser (Life Technologies, Carlsbad, CA). Chromatograms were analyzed using Sequencher (Gene Codes, Ann Harbor, MI). Bio-Rad Image Lab Software 6.0 was used for PCR band densitometry.

### Western blot analysis

Keratinocytes were lysed in protein extraction buffer (50 mM Tris-HCI, pH 7.5, 100 mM NaCl, 1% Nonidet P-40, 4 mM EDTA) containing proteinase inhibitors cocktail (Complete Mini, EDTA-free; Roche Diagnostics, Mannheim, Germany). Lysates were incubated for 30 minutes on ice and centrifuged at 15,000×g for 30 minutes at 4°C. Supernatants were collected and protein concentrations were measured using the Bradford assay (BioRad, Hercules, CA). For each sample, 40 µg of total protein was resolved on NuPAGE® Novex 3-8% Tris-Acetate gel electrophoresis (Invitrogen, Carlsbad, CA) and electrotransferred to nitrocellulose membranes (Invitrogen, Carlsbad, CA). For type VII collagen analysis, blots were probed with a monospecific polyclonal anti C7 antibody (a generous gift from Dr A. Nystrom; University of Freiburg). An antibody against GAPDHA antibody against Vinculin was used as a loading control. Visualization was performed by incubating with HRP-conjugated anti-rabbit antibody (Amersham, Burlington, MA) and West Pico Chemiluminescent Substrate (Pierce, Rockford, IL).

### Immunofluorescence and immunohistochemical staining.

For immunofluorescence detection of C7 in keratinocytes, cells grown on glass coverslips were fixed in methanol/acetone (1:1) for 10 minutes at -20ºC. After washing three times in phosphate-buffered saline (PBS) and once in PBS with 3% bovine serum albumin (BSA) (Sigma Aldrich, St Louis, MO) for 30 minutes, cells were incubated with monospecific polyclonal anti C7 antibody at 1:5000 dilution. Secondary antibody (AlexaFluor488, Invitrogen, Carlsbad, CA) was used at 1/1000 dilution. After the final washing step in PBS, preparations were mounted using Mowiol (Hoechst, Somerville, NJ) mounting medium and DAPI 20µg/ml (Sigma Aldrich, St Louis, MO) for nuclei visualization. Immunoperoxidase detection of C7 in paraffin-embedded, formalin-fixed sections was carried out with proteinase K antigen retrieval as described 33.³³. Immunoperoxidase staining for human involucrin and p63 was performed using rabbit SY5 monoclonal antibody (Sigma) and 4A4 monoclonal antibody respectively on paraffin sections without antigen retrieval. The ABC peroxidase kit (Vector) with Diaminobenzidine as a substrate was used to developing reagent.

### Electron Microscopy.

Specimens of ca. 0.4 x 0.3cm were fixed for at least 2h at room temperature in 3% glutaraldehyde solution in 0.1M cacodylate buffer pH 7.4, cut into pieces of ca. 1mm³, washed in buffer, postfixed for 1 h at 4ºC in 1% osmium tetroxide, rinsed in water, dehydrated through graded ethanol solutions, transferred into propylene oxide, and embedded in epoxy resin (glycidether 100). Semi-thin and ultrathin sections were cut with an ultramicrotome (Reichert Ultracut E). Ultrathin sections were treated with uranyl acetate and lead citrate, and examined with an electron microscope (JEM 1400) equipped with a 2k CCD camera (TVIPS).

### Generation of skin equivalents, grafting onto immunodeficient mice and graft analysis.

Animal studies were approved by our institutional animal care and use committee according to national and European legal regulations.

Gene edited keratinocytes were seeded on fibrin dermal equivalents containing RDEB fibroblasts null for C7 expression prepared as previously described ³⁴. Bioengineered skin equivalents were grafted onto the back of 7-week-old female immunodeficient mice (nu/nu, NMRI background) purchased from Elevage-Janvier (France) as previously described ³⁰. Grafting was performed under sterile conditions and mice were housed in pathogen-free conditions for the duration of the experiment at the CIEMAT Laboratory Animals Facility (Spanish registration number 28079-21 A). Animals were housed in individually ventilated type II cages, with 25 air changes per hour and 10 kGy gamma irradiated soft wood pellets as bedding. All handling was carried out under sterile conditions, and all experimental procedures were according to European and Spanish laws and regulations. Mice were sacrificed at 10 weeks post grafting and grafts harvested for skin histology, immunohistochemistry analyses and electron microscopy studies.

HDR-based corrected polyclonal keratinocytes were seeded on fibrin dermal equivalents containing RDEB fibroblasts null for C7 expression prepared as previously described 34. Bioengineered skin equivalents were grafted onto the back of 7-week-old female immunodeficient mice (nu/nu, NMRI background) purchased from Elevage-Janvier (France) as previously described 30. Grafting was performed under sterile conditions and mice were housed in pathogen-free conditions for the duration of the experiment at the CIEMAT Laboratory Animals Facility (Spanish registration number 28079-21 A). Animals were housed in individually ventilated type II cages, with 25 air changes per hour and 10 kGy gamma irradiated soft wood pellets as bedding. All handling was carried out under sterile conditions, and all experimental procedures were according to European and Spanish laws and regulations. Mice were sacrificed at different time points post grafting and grafts harvested for skin histology, immunohistochemistry analyses and electron microscopy studies.

### In vivo skin fragility test

A suction device developed in our laboratory was set up to exert a negative pressure of 10±2 kPa on a 3mm diameter area for 5 minutes to induce blister formation onto human skin grafts regenerated in immunodeficient mice 12 weeks after grafting. Two mice bearing grafts from unedited and two from sg2+sg3 RNP-treated keratinocytes were used. Suction was applied on two different sites for each graft. Before applying suction, to promote blister formation, an incandescent light bulb was set on top of the graft area approximately 2 cm away for 2 minutes 35. After that, the bulb was kept on for the entire duration of the experiment. The suctioned area was photographed 10 minutes after suctioning and excised for histological analysis.

A suction device developed in our laboratory was set up to exert a negative pressure of 10±2 kPa on a 3mm diameter area to induce blister formation onto human skin grafts regenerated in immunodeficient mice 10 weeks after grafting. Before applying suction, to promote blister formation, an incandescent light bulb was set on top of the graft area approximately 2 cm away for 2 minutes ³⁵. After that, the bulb was kept on for the entire duration of the experiment. The suctioned area was photographed 10 minutes after suctioning and excised for histological analysis.

### Results

### CRISPR/Cas9 RNP complex delivered into primary RDEB cells achieved highly efficient indel generation

To demonstrate the ability of indel generation of a sgRNAs modified guide, we evaluated the cutting efficiency of the 'sg2' guide, used in a NHEJ-based double-guide strategy in our previous work (Bonafont et al. 2019), due to the good efficiency and biosafety showed. In this case, instead of a crRNA:tracrRNA molecule for RNP complex, we tested a chemically-modified sgRNA (Synthego, CA).

This guide is targeting intron 79, very close to the pathogenic exon 80 (Figure 1A). We eliminated in our donor template construct the sequence of intron 79 to have no PAM sequence in order to avoid NHEJ events after HDR repairing events.

Sg2 was electroporated under code CM137 conditions of Amaxa 4D nucleofector platform with CRISPR/Cas9 system as RNP complex in primary RDEB keratinocytes. The ability of indel generation was assessed by TIDE analysis of NHEJ events in the target region, achieving 82.6% and 90.8% in each technical replicate in primary keratinocytes (figure 1B).

### AAV serotype testing for primary keratinocytes transduction and HDR-based RDEB correction in primary cells

AAV has been shown as very efficient and safe vectors for donor template delivery in HDR-based gene editing on different cell types, as CD34+ cells or iPSC, with promising therapeutic benefits on untreatable diseases.

So, to evaluate the AAV transduction efficiency in primary keratinocytes, we evaluated a wide collection of AAV serotypes (AAV 1,2,5,6,7,8,9 and DJ; figure 2A) packing a GFP-based construct to select the best performing subtype. GFP expression of the different AAVs was evaluated by flow cytometry, revealing AAV6 as the most powerful serotype to reach keranitocytes transduction. Therefore, we packaged our constructs in AAV vectors with serotype 6.

After optimization of the transduction protocol, we electroporated RDEB primary keratinocytes with the sg2 sgRNA as RNP and then, they were transduced with the AAV6-carrying donor template. We tested two different donor designs, one with symmetrical and another one with asymmetrical homology arms. Symmetrical donor covers E74 to E84 and asymmetrical one covers E77 to E88 of *COL7A1* gene. We analyzed by PCR, TOPO cloning and Sanger sequencing the repairing events occurred and we found close to 40% of HDR frequency, missing 179 and correcting c6527insC mutation in E80 (Figure 2B), and no difference in HDR efficiency was observed between both donors.

### De novo C7 expression after HDR-based correction in RDEB primary keratinocytes

Highly efficient gene correction in *COL7A1* should restore C7 expression in a high percentage of cells within the edited keratinocytes bulk population. Therefore, we analyzed C7 expression by immunofluorescence and Western blot in RDEB keratinocytes electroporated with the sg2 RNP and transduced with the two different donor template carrying-AAV6. The number of C7-expressing cells detected by immunofluorescence analysis (Fig. 3) matched the observed HDR frequency shown by PCR and Sanger sequencing (Fig. 2). Accordingly, western blot analysis from cellular extracts further demonstrated high expression of restored C7 with the two types of AAV6 (symmetrical and asymmetrical), similar to the healthy donor sample. This suggests we could use both donors to treat RDEB patient cells, increasing the population of RDEB patients that could undergo this gene therapy treatment.

### Long-term engraftment testing of gene corrected RDEB keratinocytes

The high percentage of cells expressing C7 after AAV6 and RNP treatment should be enough to achieve skin adhesion restoration. To assess the grade of healthy skin regeneration potential, healthy, non-treated and gene edited bulk **keratinocytes population** combined with C7 nule fibroblasts, were used to generate **skin equivalents** that were transplanted onto nude mice. H&E histological analysis showed normal skin arquitecture in grafts from healthy and gen edited keratinocytes, while some blisters were observed in grafts from untreated patient 1. Immunohistochemistry C7 detection showed no C7 expression in regenerated tissue from non-treated keratinocytes from RDEB Patient 1 (Figure 4, A). On the other hand, grafts from RDEB keratinocytes after AAV6 plus RNP treatment, P1 edited keratinocytes, revealed **C7 expression** in the basement membrane of the regenerated skin (Figure 4, B), in a similar way to grafts regenerated from healthy donor keratinocytes (Figure 4,C). All the tissue samples showed correct suprabasal human involucrin expression, proving normal epidermal human differentiation (Figure 4, D,E y F).

### HDR-based correction in a RDEB patient with mutation in E79

Donor template covers a higher amount of exons within COL7A1, making feasible gene correction at different points of the gene. Therefore, after showing relevant correction efficiency in exon 80-bearing mutation patient cells (Patient 1), we tested the exon 79-80 fusion strategy to correct a RDEB patient carrying a mutation in E79 in homozygosis (Patient 2; P2). We only tested AAV6-containing symmetrical arms for this transduction. Genotyping showed similar HDR-based correction ratios by PCR, compared to the previous treated P1 (Fig. 5). Also, we assessed C7 expression restoration by immunofluorescence of RDEB P2 treated cells, showing a significant percentage of positive C7 cells in the bulk edited population.

### CD34⁺ and MSC gene edited cells as a cell source for bone-marrow transplantation in EB

Recently, HSCT has been considered as a therapeutic option for EB treatment. Although HSCT offers a benefit to ameliorate the symptoms, it has some complications. Allogenic HSCT containing gene corrected cells could overcome this barrier and offer a safer therapeutic solution. CD34⁺ and MSCs are the main stem cell types at the bone marrow, so due to this, we targeted cord blood-isolated CD34⁺ and MSC cells from three healthy donors with the RNP plus AAV6 strategy to test the potential of our approach targeting another relevant cell types for RDEB treatment.

After 5 days, we analyzed CD34+ gene correction by PCR and we found similar gene correction ratios than in HK P1 treated cells with the same Donor template containing-AAV6 (Figure 6). For CD34+ cells, we tested two different MOIs, 5K and 10K, and no difference in HDR-based correction efficiency was observed. Moreover, comparing different cell donors, no difference of HDR events was showed. In a similar way, MSCs from three healthy donors showed percentage of precise correction close to 50%, in all the cell donors tested. No remarkable difference in editing efficiency was observed among each cell donor, supporting the sturdiness of this genome editing approach. This study provides a proof-of-concept that bone marrow stem cells are suitable for gene correction treatment with the strategy proposed and they could offer a different and potential benefit for EB treatment.

## Claims

1. An *in vitro* method for inducing a stable gene modification of a target nucleic acid comprising one or more mutant alleles comprising disease-causing mutations of the COL7A1 gene via homologous recombination in primary cells selected from the list consisting of keratinocytes or skin fibroblasts, wherein the method comprises introducing into the primary cells: (a) a modified single guide RNA (sgRNA) comprising a nucleotide sequence that is complementary to the target nucleic acid and a nucleotide sequence that interacts with a CRISPR-associated protein (Cas) polypeptide, wherein the RNA component can be two individual RNA molecules (crRNA and tracrRNA) or a single RNA molecule (sgRNA); (b) a Cas polypeptide, an mRNA encoding a Cas polypeptide, and/or a recombinant expression vector comprising a nucleotide sequence encoding a Cas polypeptide, wherein the modified sgRNA, or crRNA and tracrRNA components provided separately, guide the Cas polypeptide to the target genomic sequence to be corrected; and (c) a donor template DNA homologous to the genomic sequence comprising the mutation site to be repaired, carried by serotype 6 adeno-associated viral vectors (AAV-6) or carried by serotype 1 adeno-associated viral vectors (AAV-1);
wherein, the stable gene modification of the target nucleic acid, based on the replacement of one or more mutant alleles comprising the disease-causing mutations of the COL7A1 gene (target nucleic acid), occurs by providing AAV-6 or AAV-1 vectors carrying the correction donor templates comprising wild-type alleles corresponding to the mutant alleles.

2. The method of claim 1, wherein the Epidermolysis Bullosa disease-causing mutations are recessive Dystrophic Epidermolysis Bullosa (RDEB) disease-causing mutations.

3. The method of any of claims 1 or 2, wherein the one or more mutant alleles comprising the disease-causing mutations of the COL7A1 gene (target nucleic acid) are located in any of exons 73, 74, 75, 80 or 105 of the COL7A1 gene, and these mutations are repaired by using a correction donor template comprising the wild type exons 73, 74, 75, 80 or 105 of the COL7A1 gene.

4. The method of any of claims 1 to 3, wherein the primary cells are isolated from a mammal, preferably from a human subject, prior to introducing the modified sgRNA, the Cas polypeptide, and the AAV-6 or AAV-1 vector carrying the homologous donor template into the primary cells.

5. The method of any of claims 1 to 4, wherein the Cas polypeptide is a Cas9 polypeptide or a variant thereof, or a fragment thereof.

6. The method of any of claims 1 to 5, wherein the donor template does not contain the Cas recognized- Protospacer Adjacent Motif (PAM) sequence, or wherein the donor template does not contain one or more intronic regions of the targeted nucleic acid.

7. The method of any of claims 1 to 6, wherein the RNA component and/or the Cas polypeptide are introduced into the primary cells by electroporation and wherein optionally the AAV-6 or AAV-1 vector carrying the homologous donor template is introduced into the primary cell by transduction.

8. The method of any of claims 1 to 7, wherein the RNA component and the Cas polypeptide are incubated together to form a ribonucleoprotein (RNP) complex prior to introducing into the primary cell and wherein optionally the RNP complex and the homologous donor AAV-6 or AAV-1 vector are sequentially introduced into the primary cells.

9. A primary cell or cell population comprising the stable gene modification of the target nucleic acid obtained or obtainable by the method of any of claims 1 to 8.

10. The cell population comprising the primary cells in accordance with claim 9, wherein said population includes at least about 30% primary keratinocytes having the stable gene modification of the target nucleic acid.

11. A pharmaceutical composition comprising the primary cells or cell population of any of claims 9 or 10.

12. *In vitro* use of a kit comprising (a) a modified single guide RNA (sgRNA) comprising a nucleotide sequence that is complementary to the target nucleic acid and a nucleotide sequence that interacts with a CRISPR-associated protein (Cas) polypeptide, wherein the RNA component can be two individual RNA molecules (crRNA and tracrRNA) or a single RNA molecule (sgRNA); (b) a Cas polypeptide, an mRNA encoding a Cas polypeptide, and/or a recombinant expression vector comprising a nucleotide sequence encoding a Cas polypeptide, wherein the modified sgRNA, or crRNA and tracrRNA components provided separately, guide the Cas polypeptide to the target genomic sequence to be corrected; (c) an adeno-associated viral (AAV6) or AAV-1 vector comprising a recombinant donor template comprising two nucleotide sequences comprising two non-overlapping, homologous portions of the target nucleic acid, to undergo homologous recombination,
in a method for inducing a stable gene modification of a target nucleic acid comprising one or more mutant alleles comprising disease-causing mutations of the COL7A1 gene via homologous recombination in primary cells selected from keratinocytes or fibroblasts obtained from a subject, preferably a human subject.

13. The pharmaceutical composition of claim 11, or the primary cells or cell population of any of claims 9 or 10, for use in a method of preventing or treating Epidermolysis Bullosa, preferably recessive Dystrophic Epidermolysis Bullosa (RDEB), in a subject in need thereof, the method comprising administering to the subject the said composition in a sufficient amount to correct a mutation in the target nucleic acid that is associated with the disease, to prevent the disease or ameliorate one or more symptoms of the disease.

14. In *vitro* use of the primary cells or cell population of any of claims 9 or 10, to manufacture skin equivalents.

15. Skin equivalents obtainable or obtained according to claim 14, for use in the treatment of Epidermolysis Bullosa, particularly the recessive dystrophic subtype (RDEB).
